(19) Europäisches Patentamt
European Patent Office
Office européen des brevets

(11) Publication number: **0 363 337 A1**

# (12) EUROPEAN PATENT APPLICATION

(21) Application number: 89850292.7

(22) Date of filing: 06.09.89

(51) Int. Cl.5: A61K 31/19 , A61K 9/08 , A23L 1/30 , A23K 1/16

The title of the invention has been amended (Guidelines for Examination in the EPO, A-III, 7.3).

(30) Priority: 07.09.88 SE 8803144

(43) Date of publication of application: 11.04.90 Bulletin 90/15

(84) Designated Contracting States: ES GR

(71) Applicant: KABIVITRUM AB
S-112 87 Stockholm(SE)

(72) Inventor: Lindgren, Svante
Lättsta Skepptuna
S-195 00 Märsta(SE)
Inventor: Sandberg, Göran
Björkskog
S-762 00 Rimbo(SE)
Inventor: Enekull, Ulla
Västerasgatan 6 II
S-113 43 Stockholm(SE)
Inventor: Werner, Tom
Nysätravägen 5
S-131 33 Nacka(SE)

(74) Representative: Onn, Thorsten et al
AB Stockholms Patentbyra Zacco & Bruhn
P.O. Box 3129
S-103 62 Stockholm(SE)

## (54) Energy substrate containing hydroxycarboxylic acid.

(57) The invention relates to an energy substrate for nutrient administration to mammals in a catabolic state, and includes at least one hydroxy carboxylic acid of the general formula

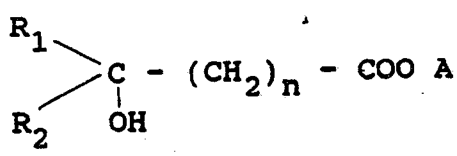

wherein $R_1$ is hydrogen or straight or branched alkyl or alkenyl and $R_2$ is straight or branched alkyl or alkenyl, wherein when $R_1$ is hydrogen, $R_2$ will always be branched alkyl or alkenyl, wherein $R_1$ and $R_2$ together contain from 2 to 6 carbon atoms, n is zero or 1, and A is hydrogen or a pharmaceutically acceptable metal cation.

EP 0 363 337 A1

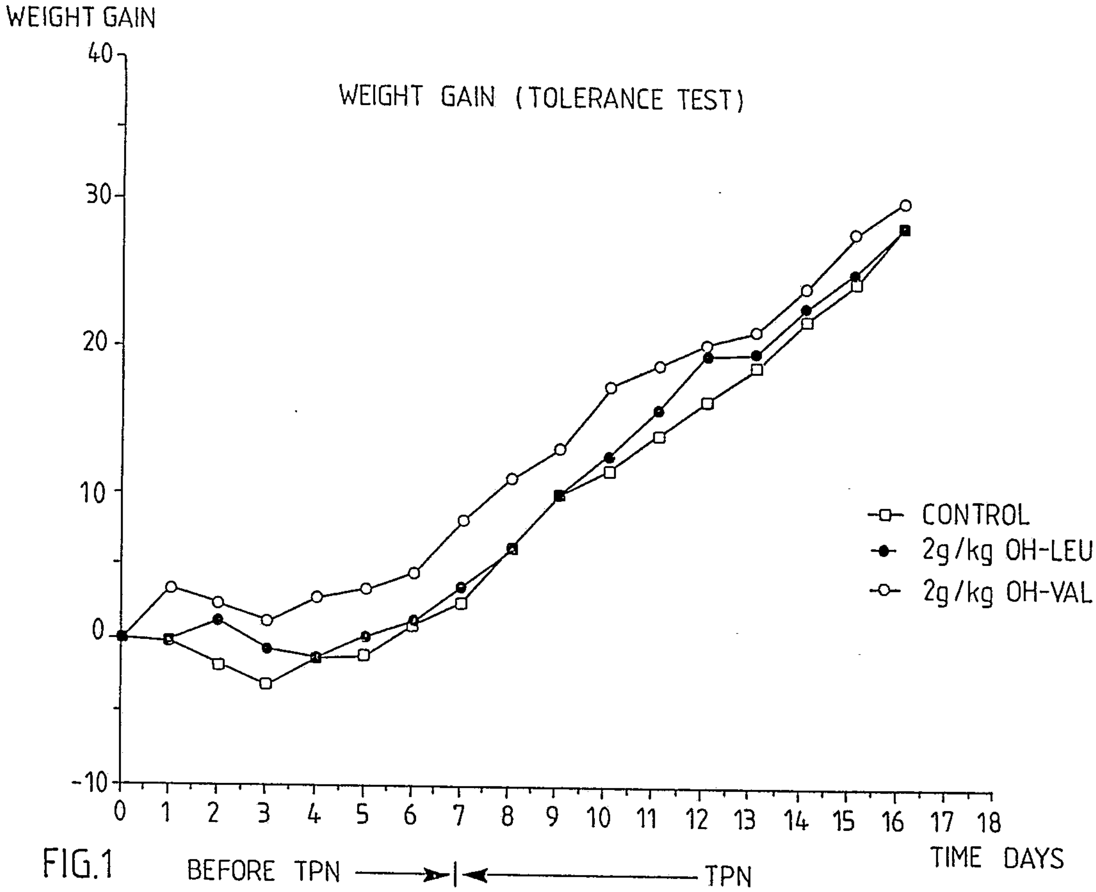
WEIGHT GAIN
40
30
20
10
0
-10
WEIGHT GAIN (TOLERANCE TEST)
CONTROL
2g/kg OH-LEU
2g/kg OH-VAL
0 1 2 3 4 5 6 7 8 9 10 11 12 13 14 15 16 17 18
TIME DAYS
BEFORE TPN
TPN

FIG.1

The present invention relates to a pharmaceutical energy substrate and pertains to the field of clinical nutrition. This field is concerned with all forms of enteral and parenteral nutrition of mammals.

Those groups of patients subjected to clinical nutrition in its various forms often have a poor nutrition status. This status may be the cause of serious trauma, such as deep and extensive burns, surgical trauma, serious body injuries, various forms of cancer or sepsis. Other groups comprise patients who are unable to eat, for physiological, anatomical or other reasons, for instance unconsciousness. In the case of serious trauma, however, the metabolic picture is complicated, because of the poor utilization of glucose in the peripheral tissues. Although the glucose and insulin levels of the blood increase, no energy, or relatively little energy is gained thereby. A so-called peripheral glucose intolerance with insulin resistance has arisen (Gump F.E., Long C., Killian P and Kinney J., M.,; J. TRAUMA 14(5): 378-388, 1974. / Black P.R., Brooks D.C., Bessey P.Q., Wolfe R.R. and Wilmoore D.W. ANN.SURG. 196(4); 420-435, 1982 / Drobney E.C., Abramson E.C. and Baumann G.J.CLIN. ENDOCRINOL.METAB. 58(4):710, 1984 etc.).

In the case of glucose intolerance, the oxidation of fat is also reduced and the ketone body metabolism is unchanged, which means that the total energy recovery is impaired (Ryan et al METAB. 23(11), 1081 1974., / O'Donnel T.F., Blacburn G.L. "Proteolysis associated with a deficit of periferal energy fuel substrates in septic man. SURGERY 80; 192-200, 1972). In a situation such as this, the body mobilizes different body proteins as an energy source, which results in a more or less extreme protein catabolic situation.

Many attempts have been made to normalize the clinical picture and to restore the concentration of branched amino acids in various stress conditions, but with varying results.

As before mentioned, trauma and sepsis are accompanied by glucose intolerance and insulin resistance. This leads to high plasma concentrations of glucose, lactate, pyruvate and alanine and also to a pronounced change in energy conversion in the muscles. Normal test subjects will exhibit the release of leucine, valine, asparagin, asparaginic acid, glutamine and glutamic acid, (5$\mu$mol/$m^2$ and minute). This release, however, increases with sepsis and some traumatic conditions by about 80% (8-9$\mu$mol/$m^2$ and minute).

Plasma exhibits low concentrations of ketone bodies, branched amino acids and albumin, and high concentrations of triglycerides, aromatic amino acids (tryptophan, phenylanaline and tyrosine), methionine and histodine, and also increasing concentrations of circulating lactate and pyruvate. The patient will die, unless this pathological picture is normalized (Birhahn R.H. and Border J.R. Am. J. Clin. Nutr. 31., 436-441, 1978 Birkhahn R.H. and Border J.R. JPEN 5(1), 1981.)

Essentially the branched amino acids, and then primarily leucine, are oxidized in the peripheral protein depôts (the muscles), although the remaining branched amino acids isoleucine and valine are also oxidized in these depôts. This means, for example, that the pool of branched amino acids of this category of patient will be gradually depleted. This results, in turn, in reduced protein synthesis in the liver.

The liver utilizes different amino acids as energy substrates to varying degrees, primarily alanine. Splanchnicus (walls of the intestines, etc.) uses mainly glutamine as an energy source. (Ryan N.T., Blackburn G.L. and Clowes Jr. G.H.A. METAB 23(11); 1081, 1974. / O'Donnel Jr. T.F., Clowes Jr. G.H.A., Blackburn G.L., Ryan N.T. Bernotti P.N. and Miller J.D.B. SURGERY 80(2); 192-200, 1976. / Duke Jr. J.H. Jorgensen S.B., Broell J.R., Long C.L. and Kinney J.M. SURGERY 68(1); 168174, 1970).

In summary, the following metabolic conditions exist in trauma and sepsis:

a Increased proteolysis of muscle proteins and increased oxidation of amino acids.

b Hyperglycemia. (The lever continues to produce glucose despite high insulin levels).

c Increased peripheral take-up of glucose.

d Increased production of pyruvate.

e Reduced oxidation of pyruvate, as a result of restraint in the pyruvate metabolism.

f Increased production of lactate, alanine and pyruvate.

g Decreased sensitivity to the effect of insulin on the glucose metabolism in the muscle cells (insulin resistance).

h A decrease in the plasma concentration of glutamine is also present in many trauma situations.

The glucose intolerance with insulin resistance has been established as a postreceptoric defect (Black P.R., Brooks O.C., Bessey P.Q., Wolfe R.R. and Wilmore D.W. ANN.SURG. 196(4) 420-435, 1982 / Drobny E.C., Abramson and Baumann G.J. DLIN.ENDOCR. METAB. 58(4) 710, 1984). This is characterized in that glucose is metabolised solely to and including pyruvate. Blocking either takes place at the pyruvate dehydrogenase and/or the carrier. The end result of this is that the mitocondria matrix receives smaller quantities of energy substrate from glycolysis, for conversion to acetyl-CoA. Since the glucose requirement of the cell. cannot be satisfied, the insulin concentration again increases. The insulin levels are, in this way, increased without the cell receiving more energy from glucose. A diabetes-like situation has arisen.

When taken together, the above facts illustrate the complex metabolic conditions for the branched amino acids in stress situations. The net result is disturbed energy conversion, where only one available substrate (BCAA, branched amino acids) is converted to energy, although even then with certain limitations.

Certain things can be observed when administering total parenteral nutrition to this category of patient:

1. The administration of solely glucose, intravenously, in the case of glucose intolerance has only negative effects in the main.

2. The administration of fat, intravenously, in the case of, e.g., sepsis, can in certain cases be directly contraindicative. A liver insufficiency usually exists with subsequent unchanged fat metabolism.

3. It is necessary to find a glucose substitute in the aforesaid stress situation with glucose intolerance.

4. The administration of branched amino acids gives a varying result and can, in certain instances, be stressful in itself. Furthermore, oxidation of these amino acids results in an increase in the concentrations of nitrogen metabolites in the blood, a circumstance which must be strongly questioned in view of the fact that in the case of sepsis with, for instance, an endo-toxin shock with impaired liver function, the renal secretion of e.g., ammonia is impaired due to the lowered blood pressure, which must be considered highly toxic to the brain.

5. A possible, new substrate should be capable of replacing amino acids, glucose and fat from an energy aspect, even under normal metabolic conditions.

The pyruvate can, to some extent, disturb the dehydrogenase for the branched alpha-keto acids, via a functional return effect. It has also been found (in vitro) that high concentrations of pyruvate can block the formation of glutamic acid from alpha-keto glutaric acid in the transamination of branched amino acids. This discovery is supported by the fact that circulating blood concentrations of glutamine are radically lowered in trauma. (Clowes Jr. G.H.A., Randall H.T. and Chac J. JPEN 4(2) 195-205; 1980. / Van Hinsbergh V.W., Veerkamp J.H., Engelen P.J.M. and Ghjisen W.J. BIOCHEM. MED. 20, 115-124, 1978; / Buse M.G., Biggers J.F., Karen H.F. and Buse J.F.; J. of BIOL.CHEM. 247 8085-96, 1972.). The pyruvate dihydrogenase and/or the pyruvate carrier, however, can be inhibited competitively, to some extent, by alpha-keto leucine (to a lesser degree by alpha-keto isoleucine and alpha-keto valine), which can be shown in in-vitro experiments.

In an in-vitro experiment on rat liver cells, the decarboxylation rate, however, was stimulated by branched alpha-keto acids to an extent which was from 2-9 times faster than the stimulation rate achieved with corresponding branched amino acids. An advantage is gained when branched keto acids can replace glucose partially. No experiments with branched keto acids as an energy-boosting source have previously been carried out. Instead, experiments with branched keto acids have been intended for the administration of nitrogen-free amino acid substitutes, for instance to uremics and those suffering from liver diseases (DE Patent No 2335215, 2335216; US Patent No 4,100,161, 4,100,160; EP O 227 545).

It has been difficult in practice, however, to produce and store solutions which contain branched keto acids in free form, since these acids have extremely poor stability. Acids bound to different carriers or substrates, such as metals, alkali metals and alkaline-earth metals have also shown poor stability. One solution to the problem of the poor stability and durability of the keto acids in storage is to use a concentrated solution which has been sterile-filtered and frozen. Prior to use, the keto-acid concentrate is thawed and diluted to the correct concentration. The significantly more durable branched alpha-hydroxy homologues have also been used. These homologues, however, have been used solely as nitrogen-free amino acid substitutes (German Patent No. 23 35 216 etc.)

The present invention removes the aforesaid drawbacks and provides a stable energy substrate for nutrient supply, which is converted in the body to the keto acids desired.

In accordance with the invention there is provided an energy substrate for administration to a mammal in a catabolic state, the energy substrate being characterized in that it includes at least one hydroxy carboxylic acid of the general formula

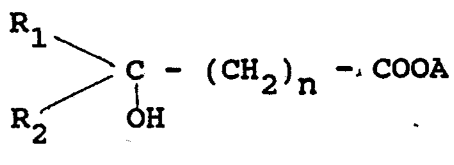

where $R_1$ is hydrogen or a straight or branched alkyl or alkenyl, and $R_2$ is a straight or branched alkyl or alkenyl, wherein when $R_1$ is hydrogen, $R_2$ is always a branched alkyl or alkenyl, and $R_1$ and $R_2$ together include from 2 to 6 carbon atoms, n is zero or 1, and A is hydrogen or a pharmaceutically acceptable metal cation.

Preferably, n has a zero value, so that the hydroxy group will always be present in the alpha-position of the carboxyl group, although it is also possible for the hydroxy group to occupy the beta-position, in which case n will be 1.

The compounds of the aforesaid general formula most preferred consist of L-α-hydroxy-isocaproic acid, alpha-hydroxy-beta-methyl valeric acid L-α-hydroxy isovaleric acid.

A pharmaceutically acceptable metal cation consists primarily of an alkali metal or an alkaline earth metal, such as sodium, potassium, calcium or magnesium, although other non-toxic metals may be used as salt formers, and then particularly iron.

The hydroxy acids used in accordance with the present invention have been found to be non-toxic at those dosages normally applicable, i.e. dosages of 1-10 g/kg body weight. The energy substrate will supply energy to an organism under normal conditions, and also under traumatic conditions, when administering a precursor to the requisite, branched keto acids. The compounds are stable, such as to enable the energy substrates to be autoclaved or pasteurized without decomposition taking place.

The energy substrates according to the invention can be administered alone to human beings and other mammals. The substrates can also be included as a component together with other compositions for parenteral nutrient administration, such as solutions of amino acids and/or carbohydrates, or fat emulsions.

In the drawings accompanying this specification. Figures 1-4 illustrate the results obtained with different experiments, in which rats were fed with the inventive energy substrate.

By branched hydroxy acids is meant here branched amino acids (e.g. leucine, isoleucine and valine), whose amino groups have been replaced with hydroxy groups. (Energy content ca 25 KJ/g.) The resultant acids can be converted to respective alpha-keto acids, by specific enzymatic reactions. The enzymes taking part in these conversions are found generally in the different organs of most mammals. The following relationship can be written:

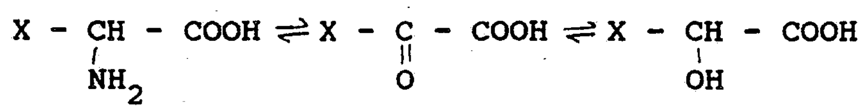

where X may be an aliphatic, branched or aromatic group.

Branched hydroxy acids normally have the L-form, although certain D-forms can be utilized by some species of mammal (Close J H, New Engl. J. Med. March 21, pages 663-667, 1974).

Experiments on branched hydroxy acids as amino-acid substitutes have shown that they are able to form respective amino acids (Chow K W and Walser M, J Nutr 105:372, 378, 1975; Pond W G et al, J Nutr 83:85-93, 1964; Chawla R K and Rudman D.J., J Clin Invest 54:271-277, 1974, etc).

Thus, the ability of branched hydroxy acids to convert to respective keto acids has been shown by several authors. The present invention utilizes this fact, insomuch as the resultant keto acid is included directly as a substrate in the oxidation of branched amino acids, by excluding the transamination step. The metabolic path of α-keto leucine is:

α-keto leucine → isovaleryl-CoA → β-crotonyl-CoA → irrev.

→ β-methyl-glutaconyl-CoA → citrate cycle → the breathing chain.

The end products of α-keto leucine are acetyl-CoA and propionyl-CoA. This last mentioned is converted to succinyl-CoA, which is also an end product of α-keto valine. Succinyl-CoA is already present in the citrate cycle and also in the breathing chain for energy recovery ($NADH + H^+$ and $FADH_2$). Branched α-hydroxy acid can be used in the aforedescribed manner as a substitute energy substrate for branched α-keto acids.

Another possibility for branched α-hydroxy acids to generate energy, is to pass directly through the inner mitochondria membrane and into matrix, where they are converted enzymatically (via a lactate-dehydrogenase) to respective α-keto acids. The reaction can be described as a shuttle in accordance with the following:

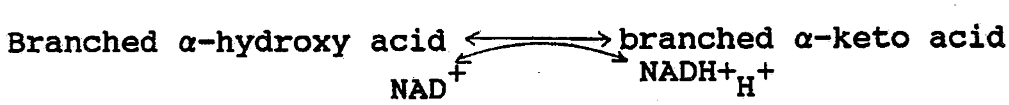


The system is described for the spermatozoa of mice (Gerez de Burgos et al, Biochem Biophys Res Comm 81(2): 644-649, 1978; Burgos C et al, Biochem J 208:413-417, 1982). This system is thought to be capable of generating energy, partly through oxidation of the α-keto acid formed and partly through the delivery of

cytosol hydrogen, which is able to enter the breathing chain directly as $NADH^{+}H^{+}$.

The inventive energy substrate can be administered in the form of an aqueous solutions containing one or more of the branched hydroxy acids in free form or in the form of the salts mentioned in the aforegoing. Such nutrient solutions will often also contain further nutrient components, such as amino acids, carbohydrates, and/or fat, in proportions adapted to those factors associated with the reasons for administering nutrient, the state of the patient and like conditions. For instance, the solutions may contain 0.1-10 percent by weight of branched hydroxy acids as salts, optionally in combination with essential and non-essential amino acids in appropriate proportions. The solutions may also contain glucose or fructose, and even a carnitine addition of 0.01-10 g/l.

The solutions may also contain fat, in the form of a fat emulsion, the amount of fat present being adapted to the clinical status of the patient. For example, small quantities of fat are administered in the case of sepsis, whereas large quantities of fat, up to 200 g/day, may be required in the case of serious trauma, such as burn injuries.

The inventive solutions may also contain other components necessary to provide a complete nutrient, such as vitamins, electrolytes and trace elements.

The solutions of the inventive energy substrate will also include conventional auxiliary substances used in solutions intended for parenteral administration. Such auxiliary substances include agents for adjusting pH and the osmotic pressure, preservatives and stabilizers, and other substances conventional to the person skilled in this art.

When the energy substrate according to the invention is to be administered together with other nutrient components, such as the abovementioned amino acid solutions, carbohydrate solutions and fat emulsions, theses can be mixed together in a solution of hydroxy acid and administered as a single composition. In some cases, however, the various components of the mixture may act detrimentally on one another, so as to render the stability of the ultimate preparation insufficient. In such cases, the various components can be administered from separate storage containers and brought into mixture with one another immediately prior to being administered to the patient, for instance in a branched pipe or some other mixing device.

The inventive energy substrate is prepared in a conventional manner, in accordance with current pharmaceutical practice. The hydroxy acid or hydroxy acids used can be readily dissolved in a water phase, and the additive substances optionally used are added either before or after the acid. Subsequent to adjusting the pH, if necessary, the mixture is sterilized, normally by autoclaving or sterile-filtering the mixture. Naturally, it is important that all of the components included in the mixture are of a pharmaceutically non-objectionable quality, and that the mixture is prepared under conditions such as to ensure that the components will not decompose or be impaired in some other way. Thus, the water used shall be sterile and free from pyrogens, and the mixture will normally be prepared under an inert gas shield, such as nitrogen gas, so as to avoid oxidation. The resultant preparation can also be stored under a protective gas environment, in conventional infusion containers.

As before mentioned the inventive nutrient substrate is primarily intended for administration to mammals, primarily human beings, in a catabolic state, where nutrient cannot be administered in a normal manner. Administration is preferably effected parenterally, and then normally intravenously. When circumstances permit, administration may be effected enterally, however, for instance, with the aid of a stomach tube.

The invention will now be described in more detail with reference to non-limiting working examples.

## Example 1

21.6 g of L-leucinic acid (L-alpha-hydroxy-isocaproic acid) and L-valinic acid (L-alpha-hydroxy-isovaleric acid) were dissolved, each per se, in a nitrogen-gas atmosphere in one liter of Vamin $^{R}$ 14 (an electrolyte-free amino acid solution containing essential and non-essential amino acids), such as to obtain two solutions, each measuring one liter. The solutions were sterile-filtered in accordance with accepted pharmaceutical standards and were pasteurized at +85°C for 2.5 hours, whereafter they were used for animal experimentation. Sprague-Dawley rat males weighing 180-190 g were implanted surgically with a central vein catheter protected by a saddle (Roos et al, 1981). Total parenteral nutrition was administered in the form of the following standard formula:

| | |
|---|---|
| Fat | 8.0 g/kg body weight and 24 hrs |
| Nitrogen | 1.2 g/kg body weight and 24 hrs |
| Total energy supplied | 1257 KJ/kg body weight and 24 hrs |
| NPC (non-protein calories) | 1098 KJ/kg body weight and 24 hrs |
| Liquid | 300 ml/kg body weight and 24 hrs |

The glucose to fat ratio was 70/30 energy percent. The percentage of energy obtained from the energy substitute was about 5 % of NPC. A total parenteral nutrition mixture (TPN) (standard formula) was obtained by mixing electrolyte-free Vamin [R] 14, glucose solution 50% and Intralipid [R] (a fat emulsion for introvenous nutrient supply) 20% in appropriate proportions, and by adding adequate quantities of electrolytes, trace elements and vitamins.

Subsequent to continuous infusion for one week (20 hours/day) the rats were divided randomly into three groups, with six animals in each group. One group was continued on the same TPN-program as earlier (the reference group). The other groups (the test groups) received 2 g L-leucinic acid and 2 g L-valinic acid in a yield corresponding to an equivalent glucose energy-quantity. In other respects, the test groups were treated with the same TPN-formula as the reference group. The experiment was continued for nine consecutive days, during which the weights of respective animals were recorded daily and their urine collected and frozen. At the end of the test period, the rats were killed by heart puncture under pentobarbital anaesthetic and an autopsy with macroscopic investigation was carried out on the rat carcases.

The weight increases recorded during the experiments are illustrated in Figure 1 of the accompanying drawings, from which it will be seen that there was no substantial weight difference between the three groups. The relative weights of a number of organs of the animals were also assayed, the result being shown in Table 1. The Table shows that no significant differences were found between the groups. Further investigations were made with respect to the water contents of body organs and carcases, and also the urine-urea nitrogen secretion per calendar day, and blood-chemical analyses were also carried out. None of these investigations showed significant differences between the different animal groups.

In summary, the results show that leucinic acid and valinic acid function as an energy substrate and that the animals experimented upon can well tolerate the intravenously adminstered dose.

Table 1

| Relative organ weights subsequent to administration of TPN over 9 days (mean value ± SD) | | | |
|---|---|---|---|
| | Group A | Group B | Group C |
| n | 5 | 6 | 6 |
| Liver (g/kg) | 30.58±1.31 | 32.06±0.39 | 31.22±2.77 |
| Spleen (g/kg) | 3.23±0.25 | 2.91±0.14 | 3.85±1.50 |
| Kidneys (g/kg) | 8.34±0.-46 | 8.90±0.35 | 8.84±0.40 |
| Lungs (g/kg) | 4.99±0.21 | 4.89±0.29 | 5.08±0.24 |
| Heart (g/kg) | 4.21±0.14 | 4.15±0.12 | 4.28±0.31 |
| Thymus (g/kg) | 2.77±0.65 | 2.32±0.24 | 2.30±0.37 |
| Group A: Reference group<br>Group B: 2 g leucinic acid<br>Group C: 2 g valinic acid | | | |

Example 2

Sprague-Dawley male rats were treated in accordance with Example 1. The animals were divided randomly into three groups, with six animals in each group. Total parenteral nutrition was administered in

accordance with a standard program:

| | | |
|---|---|---|
| Group 1 | reference solution | n=6 |
| Group 2 | 2 g L-leucinic acid per kg body weight and 24 hours | n=6 |
| Group 3 | 4 g L-leucinic acid per kg body weight and 24 hours | n=6 |

As with the previous experiment,the TPN-solutions were equicalorific, i.e. equivalent energy quantities in the form of glucose were removed from the TPN-solution in the case of those groups which received L-leucinic acid.

Subsequent to weight-increase stabilization (region 2.3-2.8 g/day and rat), rats were removed from each group for continued experiments with 14 C-(U)labelled L-alpha-hydroxy isocaproic acid.

The experiment was carried out in a system for indirect calorimetry, in which air quantity, oxygen consumption and carbon dioxide production can be recorded. Radio-active carbon dioxide deriving from the oxidation of L-leucinic acid was collected and the level of radio-activity measured with a scintillation counter (Lindmark L et al, 1986).

The results showed that L-leucinic acid is oxidized, even when the animal receives adequate energy quantities (1257 KJ/kg). No difference was observed, however, between the weight increase and body composition of the animals in the various groups, similar to the previous experiment, which indicates that L-leucinic acid metabolises instead of glucose.

Figure 2 illustrates relative urine secretion, oxidation and incorporation of L-leucinic acid during the experiment. The cumulative oxidation varied between 32 and 55% of the quantity administered, and the largest absolute amount of oxidized material was obtained in the highest dosage group, i.e. four gram L-leucinic acid per kg of body weight and day (Figures 3 and 4).

Example 3

Sprague-Dawley male rats were treated and operated on in accordance with Example 1. The animals were then divided at random into two groups, with six animals making up a reference group and eight animals making up a test group. The dosage of L-leucinic acid in the test group was adjusted to 10.0 g per kg body weight and day. The solution in which the dosages were administered was prepared in accordance with the same principles as those described in Example 1, namely by dissolving 95.6 g of L-hydroxy-leucine, were dissolved in an electrolyte-free amino acid solution containing essential and non-essential amino acids. The pH was adjusted to 5.2 by adding a solution containing NaOH and KOH. The nutrient solution was then sterile-filtered and pasturized at +85°C, for 2.5 hours. Total parenteral nutrition was administered in accordance with a standard formula:

| | |
|---|---|
| Fat | 8.0 g/kg body weight and 24 hrs |
| Nitrogen | 1.2 g/kg body weight and 24 hrs |
| Total energy supplied | 1257 KJ/kg body weight and 24 hrs |
| NPC (non-protein calories) | 1098 KJ/kg body weight and 24 hrs |
| Liquid | 300 ml/kg body weight and 24 hrs |

The ratio between glucose (incl. L-leucinic acid) and fat was 70/30 energy percent. The percentage of energy obtained from the energy substitute was 23 % of NPC. The TPN-mixture was obtained by mixing electrolyte-free Vamin® 14, glucose solution 50% and Intralipit® 20% in appropriate proportions, and by adding adequate quantities of electrolytes, trace elements and vitamins. The reference group received isocaloric TPN-regime, as did also the test group but without L-leucinic acid. The sodium and potassium were administered in equivalent amounts in both test and reference groups. The animals were infused for ten consecutive days, during which body weights were recorded and urine collected. The rats were then starved for one calendar day and then killed by heart puncture, under pentobarbital anaesthetic. An autopsy was then performed, with macroscopic investiga tion, and the various body organs weighed.

The growth of the animals during the last three days of the experiment was also determined, it being established that no essential differences existed in the growth between the animals of the various groups.

Blood, chemical analyses made subsequent to completion of the infusion period and the one day starvation period showed that no significant differences existed between the animals of the various groups. The weights of the animal organs were determined after killing the animals, but no significant differences were found.

In summary, these results show that L-leucinic acid is well tolerated at the dosages given and fulfills an energy substrate function.

## Claims

1. An energy substrate for nutrient administration to mammals in a catabolic state, characterized in that it includes at least one hydroxy carboxylic acid of the general formula

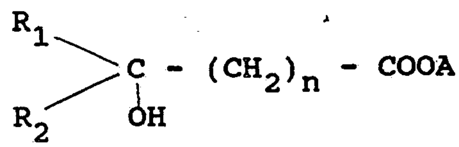

where $R_1$ is hydrogen or a straight or branched alkyl or alkenyl, and $R_2$ is a straight or branched alkyl or alkenyl, wherein when $R_1$ is hydrogen, $R_2$ will always be branched alkyl or alkenyl, $R_1$ and $R_2$ together contain from 2 to 6 carbon atoms, n is zero or 1, and A is hydrogen or a pharmaceutically acceptable metal cation, in an aqueous solution.

2. An energy substrate according to Claim 1, characterized in that n in the formula of Claim 1 is zero.

3. An energy substrate according to Claim 1 or 2, characterized in that the hydroxy acid comprises at least one of the acids L-alpha-hydroxy-isocaproic acid, alpha-hydroxy-beta-methyl valeric acid and L-alpha-hydroxy-isovaleric acid.

4. An energy substrate according to any one of Claims 1-3, characterized in that it includes from 0.1 to 10 percent by weight of at least one of the hydroxy carboxylic acids.

5. An energy substrate according to any one of Claims 1-4, characterized in that it exists in the form of an aqueous solution which also contains a plurality of amino acids.

6. An energy substrate according to any one of Claims 1-5, characterized in that it also includes metabolisable fat in emulsified form.

7. An energy substrate according to any one of Claims 1-6, characterized in that it also includes one or more of the components: carbohydrates, vitamins, electrolytes and trace elements.

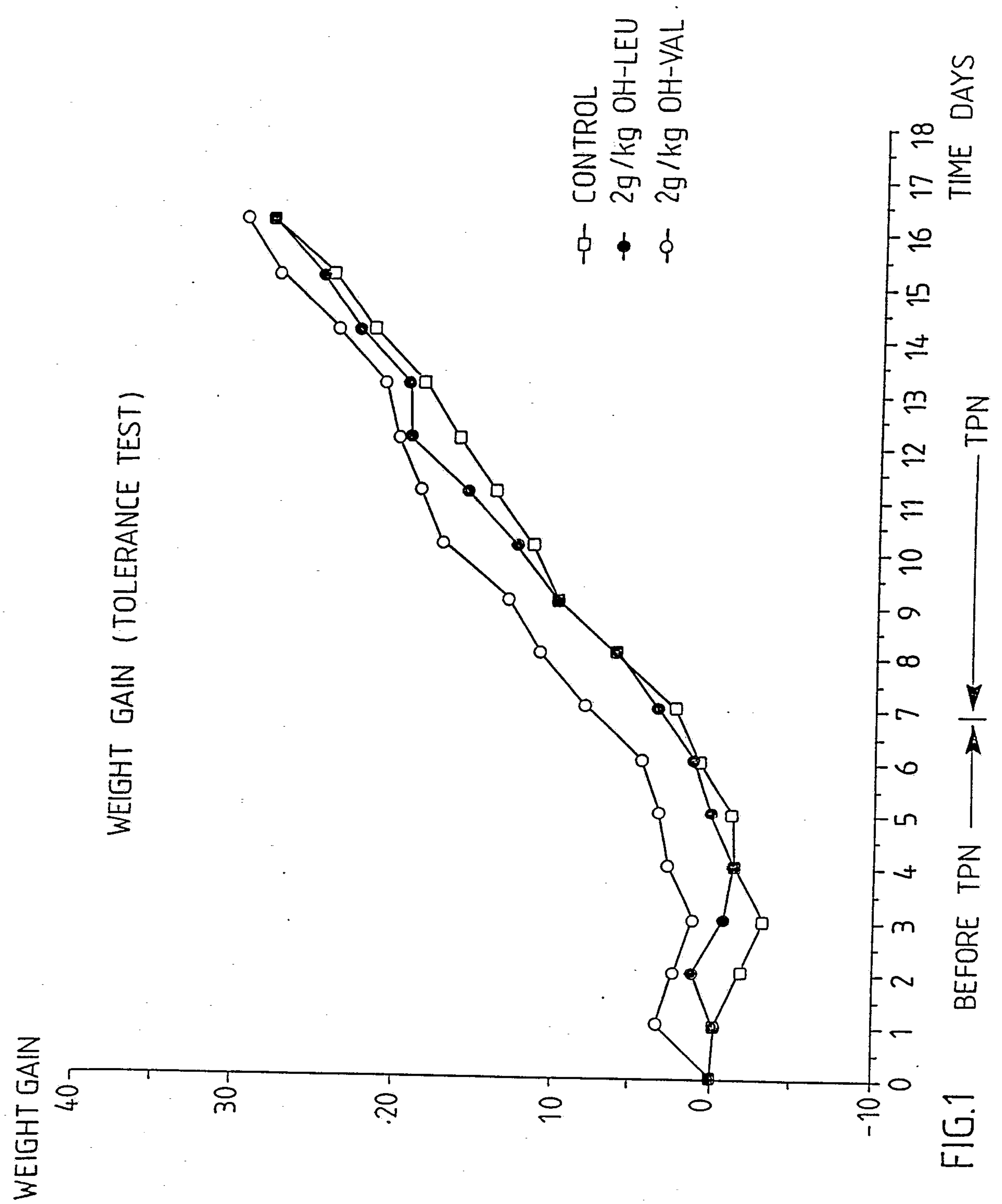
WEIGHT GAIN (TOLERANCE TEST)
WEIGHT GAIN
40
30
20
10
0
-10
0 1 2 3 4 5 6 7 8 9 10 11 12 13 14 15 16 17 18
TIME DAYS
BEFORE TPN
TPN
CONTROL
2g/kg OH-LEU
2g/kg OH-VAL

FIG.1

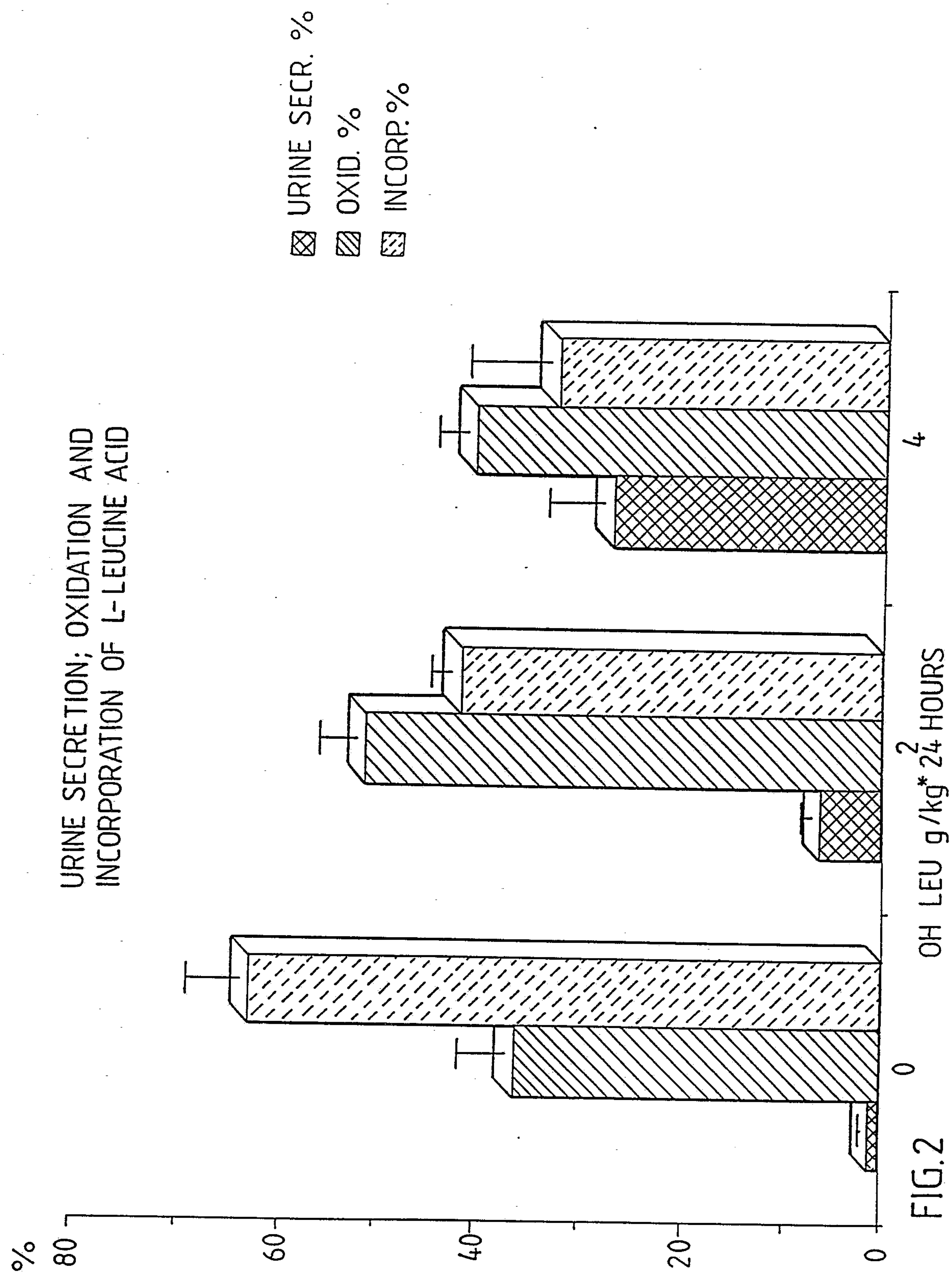
URINE SECRETION; OXIDATION AND INCORPORATION OF L-LEUCINE ACID
URINE SECR. %
OXID. %
INCORP. %
%
80
60
40
20
0
0
2
4
OH LEU g/kg*24 HOURS

FIG.2

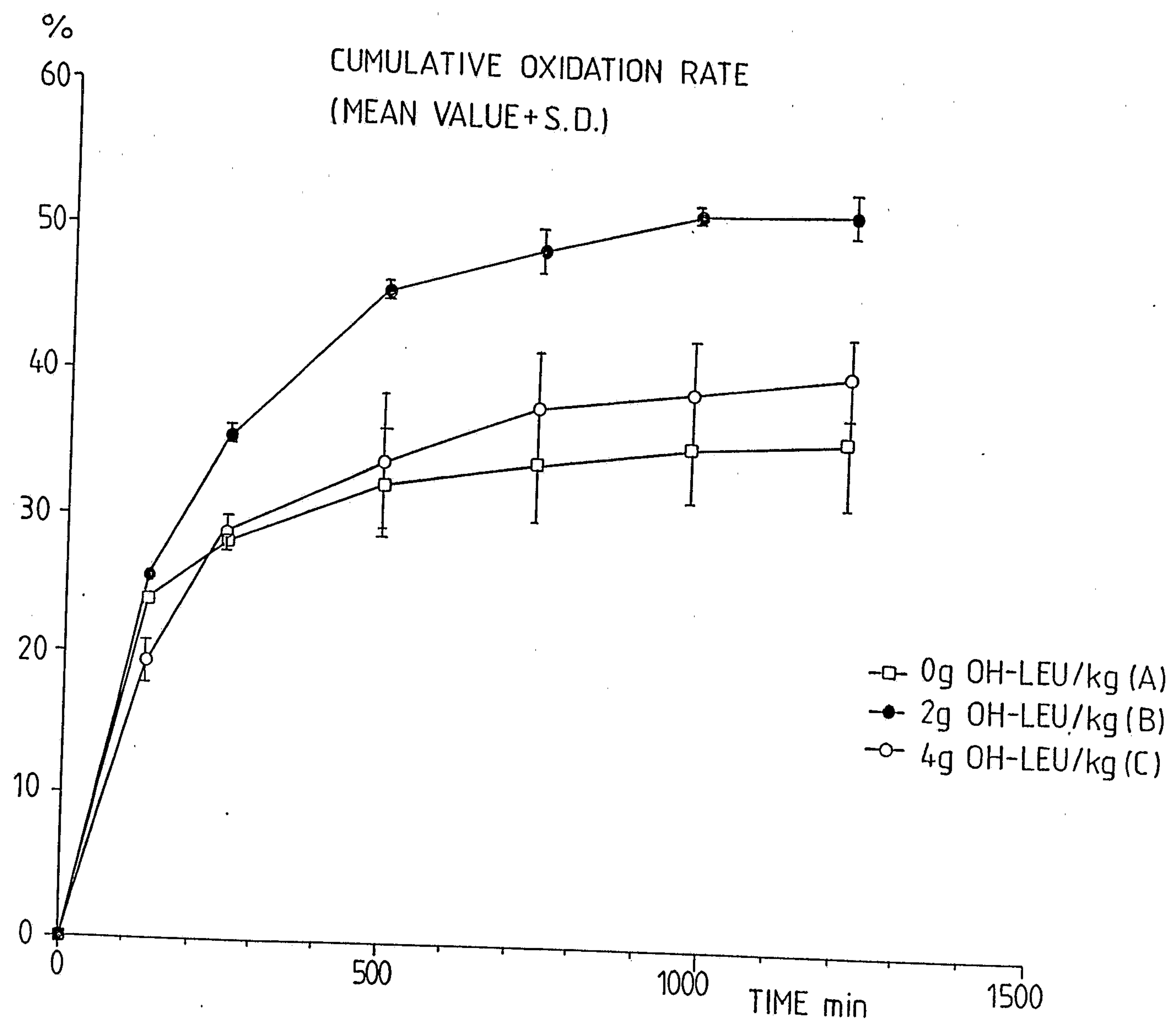
%
CUMULATIVE OXIDATION RATE
(MEAN VALUE+S.D.)
60
50
40
30
20
10
0
0
500
1000
1500
TIME min
0g OH-LEU/kg (A)
2g OH-LEU/kg (B)
4g OH-LEU/kg (C)

FIG. 3

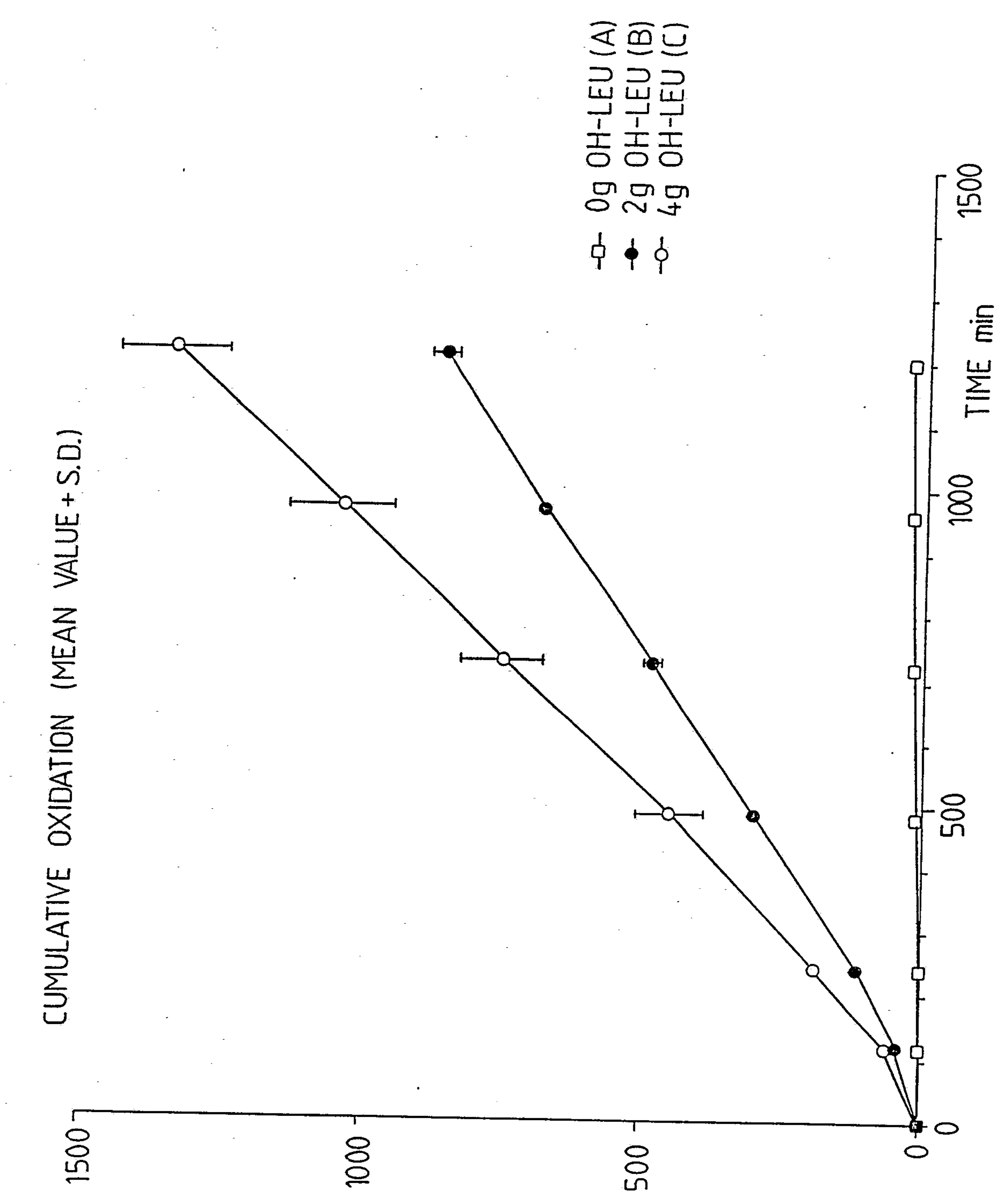
CUMULATIVE OXIDATION (MEAN VALUE + S.D.)
CUM.OX. mg/kg
1500
1000
500
0
0
500
1000
1500
TIME min
0g OH-LEU (A)
2g OH-LEU (B)
4g OH-LEU (C)

FIG.4

European Patent Office

# EUROPEAN SEARCH REPORT

Application number

EP 89 85 0292.7

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| X<br>Y | The Journal of Clinical Investigation, Vol 54, August 1974, Rajender K. Chawla and Daniel Rudman: "Utilization of x-Keto and Hydroxy Analogues of Valine by the Growing Rat"<br>*Pages 271-277*<br>--- | 1-3<br>4-7 | A 61 K 31/19, 9/08<br>A 23 L 1/30<br>A 23 K 1/16 |
| X<br>Y | J. Nutrition, Vol 83, 1964, W.G. Pond et al: "Effects of the x-Hydroxy Analogues of Isoleucine, Lysine, Threonine and Tryptophan and the x-Keto Analogue of Tryptophan and the Level of the Corresponding Amino Acids on Growth of Rats"<br>*Pages 85-93*<br>--- | 1-3<br>4-7 | |
| X<br>Y | Journal of nutrition, Vol 105,1975, Kye-Wing Chow and Mackenzie Walser: "Effects of Substitution of Methionine, Leucine, Phenylalanine, or Valine by Their x-Hydroxy Analogs in the Diet of Rats"<br>*Pages 372-378*<br>--- | 1-3<br>4-7 | TECHNICAL FIELDS SEARCHED (Int. Cl.4)<br>A 61 K<br>A 23 L<br>A 23 K |
| Y | GB-A-1 444 621 (MACKENZIE WALSER) 4 August 1976<br>--- | 1-7 | |
| Y | EP-A2-0 227 545 (THE JOHN HOPKINS UNIVERSITY) 1 July 1987<br>------ | 5 | |

The present search report has been drawn up for all claims

| Place of search | Date of completion of the search | Examiner |
|---|---|---|
| STOCKHOLM | 14-11-1989 | SIÖSTEEN Y. |

**CATEGORY OF CITED DOCUMENTS**

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document